# EUROPÄISCHE PATENTANMELDUNG

(11) **EP 2 422 752 A1**
(43) Veröffentlichungstag der Anmeldung: **29.02.2012**
(21) Anmeldenummer: 11006707.1
(22) Anmeldetag: 17.08.2011
(51) Int. Cl.: A61F 2/44

(54) **Implantantat-System für die Fusion von Wirbelkörpern**

(30) Priorität: 28.08.2010 DE 202010011948 U
(71) Anmelder: spontech spine intelligence group AG, 70182 Stuttgart (DE)
(72) Erfinder: Copp, Franz, 70184 Stuttgart (DE); Taddia, Lino, 78333 Stockach (DE); Fischer, Erik, 70619 Stuttgart (DE); Mohr, Marcus, 70839 Gerlingen (DE); Anic, Tomislav, 70599 Stuttgart (DE); Pross, Gerhard, 71039 Weil im Schönbuch (DE)
(74) Vertreter: Schwanhäusser, Gernot

(57) **Zusammenfassung**

Ein Implantat-System für die Fusion von Wirbelkörpern umfasst ein Implantat (50), das entlang einer Längsachse eine Implantatlänge (IL) hat und dazu eingerichtet ist, in ein von zwei Wirbeln (W1, W2) begrenztes Bandscheibenfach (70) eingedreht zu werden. Ferner umfasst das System eine Führungshülse (10), durch die hindurch das Implantat (50) in das Bandscheibenfach (70) eingedreht wird und die sich während des Eindrehvorgangs mit einer Schulter (36, 38) an den zwei Wirbeln (W1, W2) abstützt, und einen Bohrer (40), der in diese einführbar ist und einen Schaft (42) hat, der einen Bohrkopf (46) trägt. An dem Schaft (42) des Bohrers (40) ist ein Anschlag (48) ausgebildet, der mit der Führungshülse (10) derart zusammenwirkt, dass ein weiteres Einführen des Bohrers (40) in die Führungshülse (10) verhindert wird, sobald der Bohrer (40) relativ zu der Führungshülse (10) eine axiale Anschlagsposition erreicht. Der Bohrkopf (46) ragt in der axialen Anschlagsposition um eine Bohrlänge (BL) über die Schulter (36, 38) der Führungshülse (10) hinaus, die kleiner ist als die Implantatlänge (IL).

## Beschreibung

### HINTERGRUND DER ERFINDUNG

### 1. Gebiet der Erfindung

Die Erfindung betrifft ein Implantat-System für die Fusion von Wirbelkörpern, die ein Bandscheibenfach begrenzen. Das System umfasst ein einzudrehendes Implantat, eine Führungshülse und einen Bohrer.

### 2. Beschreibung des Standes der Technik

Bei degenerierten Wirbelsäulensegmenten besteht eine Behandlungsmöglichkeit darin, die betroffene Bandscheibe ganz oder teilweise zu entfernen und ein Implantat in das Bandscheibenfach einzusetzen. Häufig ist das Implantat mit einem Außengewinde versehen und kann dadurch in das präparierte Bandscheibenfach eingeschraubt werden. Das Implantat hält die benachbarten Wirbel auf Abstand und ermöglicht es, dass das Bandscheibenfach nach und nach verknöchert, was schließlich zu einer Fusion der benachbarten Wirbel führt.

Ein Beispiel für ein solches Implantat ist in der US 2007/0055374 A1 beschrieben. Das bekannte Implantat hat einen abgerundeten Kopf mit einem konischen Gewindeabschnitt, einen Zentralabschnitt mit großflächigen Fenstern und eine Basisabschnitt, der Befestigungsmittel für ein Eindrehinstrument aufweist. Der Basisabschnitt hat dabei in zwei orthogonalen Richtungen unterschiedliche Abmessungen. Durch Verdrehen des Implantats um 90° können somit unterschiedliche Winkel zwischen den benachbarten Wirbelkörpern eingestellt werden.

Bevor das Implantat eingedreht werden kann, muss das Bandscheibenfach in der Regel distrahiert werden. Hierzu kann beispielsweise ein System aus mehreren aufeinander abgestimmten Führungshülsen und Distraktoren verwendet werden, wie es in der noch unveröffentlichten deutschen Patentanmeldung "System zum Distrahieren eines Bandscheibenfachs" der Anmelderin beschrieben ist, die am 24.08.2010 eingereicht wurde.

Nach erfolgter Distraktion ragen zwei an den distalen Enden der Führungshülsen vorspringende Distanzzungen in das Bandscheibenfach hinein und erhalten die Distraktion aufrecht. Nach dem Entfernen des letzten Distraktors kann durch die zuletzt eingesetzte Führungshülse ein Eindrehinstrument eingeführt werden, an dessen distalem Ende das Implantat befestigt ist.

Gelegentlich kann es jedoch vorkommen, dass sich die beiden benachbarten Wirbelkörper nicht so weit distrahieren lassen, dass das Implantat in das Bandscheibenfach eingeführt werden kann. In solchen Fällen ist es zweckmäßig, das Bandscheibenfach zunächst mit Hilfe eines Bohrers aufzubohren.

Das Aufbohren des Bandscheibenfachs ist allerdings auch mit Nachteilen verbunden. Zum einen wird hartes Knochenmaterial im Bereich der Ränder der Wirbelkörper entfernt, was potenziell die Stabilität des Wirbelkörpers schwächt. Zum anderen ist das Aufbohren nicht frei von Risiken, da der Bohrer bis an die hinteren Knochenränder der benachbarten Wirbelkörper herangeführt wird. Jenseits dieser Knochenränder befindet sich empfindliches Gewebe wie Blutbahnen, weswegen Aufbohrungen in diesem Bereich eine besondere Aufmerksamkeit des Chirurgen erfordern.

Ein weiteres Problem entsteht dadurch, dass der Chirurg im Allgemeinen vor der Operation ein Implantat aussucht, nach dessen Einsetzen das Bandscheibenfach eine gewünschte Soll-Geometrie erhält. Stellt der Chirurg während der Operation fest, dass er die benachbarten Wirbelkörper nicht weit genug distrahieren kann und deswegen das Bandscheibenfach aufgebohrt werden muss, so erfordert dies in der Regel, dass dann auch ein anderes als das ursprünglich vorgesehene Implantat eingedreht werden muss. Dies erhöht die Komplexität des chirurgischen Vorgangs und auch die Zahl der Implantate, die für die Operation bereitgehalten werden muss. Beides wirkt sich ungünstig auf die Kosten der Behandlung aus.

### ZUSAMMENFASSUNG DER ERFINDUNG

Aufgabe der Erfindung ist es, ein Implantat-System mit einem einzudrehenden Implantat, einer Führungshülse und einem Bohrer anzugeben, welches die vorstehend beschriebenen Nachteile, die im Zusammenhang mit dem Aufbohren entstehen können, zumindest teilweise überwindet.

Gelöst wird diese Aufgabe erfindungsgemäß durch ein Implantat-System für die Fusion von Wirbelkörpern, das ein (vorzugsweise mit einem Außengewinde versehenes) Implantat umfasst, das entlang einer Längsachse eine Implantatlänge hat und dazu eingerichtet ist, in ein von zwei Wirbeln begrenztes Bandscheibenfach eingedreht zu werden. Das Implantat-System umfasst ferner eine Führungshülse, durch die hindurch das Implantat in das Bandscheibenfach eingedreht wird und die sich während des Eindrehvorgangs mit einer Schulter an den zwei Wirbeln abstützt. Ferner umfasst das Implantat-System einen Bohrer, der von einem proximalen, dem Chirurgen zugewandten Ende der Führungshülse her in diese einführbar ist und einen Schaft hat, der an seinem distalen, dem Chirurgen abgewandten Ende einen Bohrkopf trägt. Erfindungsgemäß ist an dem Schaft ein Anschlag ausgebildet, der mit der Führungshülse derart zusammenwirkt, dass ein weiteres Einführen des Bohrers in die Führungshülse verhindert wird, sobald der Bohrer relativ zu der Führungshülse eine axiale Anschlagsposition erreicht. In dieser axialen Anschlagsposition ragt der Bohrkopf um eine Bohrlänge über die Schulter der Führungshülse hinaus, die kleiner ist als die Implantatlänge.

Mit anderen Worten ist die vom Bohrkopf erzeuge Bohrung stets kürzer als das Implantat. Aufgebohrt wird das Bandscheibenfach somit nicht über die gesamte Implantatlänge hinweg oder gar darüber hinaus, sondern nur innerhalb eines kürzeren proximalen, d. h. dem Chirurgen zugewandeten Abschnitts.

Dahinter steht die Überlegung, dass bei vielen gängigen Implantatgeometrien auf ein Aufbohren der distalen, vom Chirurgen abgewandten Knochenränder der das Bandscheibenfach begrenzenden Wirbelkörper verzichtet werden kann. Denn dort ist der Abstand zwischen den Wirbelkörpern, wenn diese sich während der Operation in einer distrahierten Stellung befinden, ohnehin am größten. Problematisch, weil gewissermaßen "zu eng", ist vielmehr das proximale Ende des Bandscheibenfachs, denn dort ist in der distrahierten Stellung der Abstand zwischen den Wirbelkörpern in der Regel kleiner. Daher bereitet es im Allgemeinen nur dort Schwierigkeiten, den dickeren vorderen Abschnitt des Implantats in das Bandscheibenfach einzuführen.

Erfindungsgemäß wird deswegen nur dieses engere proximale Ende des Bandscheibenfachs mit Hilfe des Bohrers aufgebohrt. Der Anschlag stellt dabei sicher, dass der Bohrer nicht weiter als unbedingt nötig in das Bandscheibenfach eindringen kann. Dadurch besteht keine Gefahr, dass hinter dem distalen Ende der Wirbelkörper liegendes empfindliches Gewebe durch den Bohrer beschädigt wird. Außerdem wird durch eine kurze Bohrung die Stabilität der Wirbelkörper weniger beeinträchtigt.

Besonders vorteilhaft ist die Erfindung somit dann einsetzbar, wenn das Implantat seinen größten Durchmesser in seiner distalen, d.h. während des Eindrehvorgangs vom Chirurgen abgewandten, Hälfte hat.

Bei einem bevorzugten Ausführungsbeispiel hat das Implantat im Wesentlichen die Form eines Doppelkegels. Der Abschnitt, an dem die beiden Doppelkegel aneinander angrenzen, ist dann gleichzeitig der Abschnitt mit dem größten Durchmesser des Implantats.

Da im Allgemeinen nur die dem Chirurgen zugewandten proximalen Knochenränder der angrenzenden Wirbelkörper aufgebohrt werden müssen, kann die Bohrlänge relativ kurz sein, und beispielsweise weniger als 2/3 oder sogar 1/2 der Implantatlänge betragen.

Bei einem besonders bevorzugten Ausführungsbeispiel hat das Implantat an seinem proximalen, d. h. während des Eindrehvorgangs dem Chirurgen zugewandten, Ende einen Basisabschnitt, der entlang zweier Richtungen, die senkrecht zu der Längsachse des Implantats verlaufen, unterschiedliche Abmessungen hat.

Je nach der Winkelorientierung, in der das Implantat im Bandscheibenfach verbleibt, ergeben sich dadurch unterschiedliche Winkel zwischen den angrenzenden Wirbelkörpern.

Dies kann wiederum dazu ausgenutzt werden, einen Ausgleich für den Materialabtrag zu schaffen, der gegebenenfalls durch einen Bohrvorgang verursacht wird. Die Abmessungen entlang der zwei Richtungen sind dann derart an den Durchmesser des Bohrkopfes angepasst, dass dann, wenn mit dem Bohrer eine Bohrung erzeugt wurde und sich das Implantat in einer ersten Winkelstellung zwischen den Wirbelkörpern befindet, der Abstand und der Winkel zwischen den Winkelkörpern gleich groß ist, wie wenn mit dem Bohrer keine Bohrung erzeugt wurde und sich das Implantat in einer zweiten Winkelstellung befindet, die durch Drehung (bei orthogonalen Richtungen um 90°) aus der ersten Winkelstellung hervorgeht.

Auf diese Weise erhält der Chirurg die Möglichkeit, unabhängig davon, ob er mit Hilfe des Bohrers das Bandscheibenfach aufbohrt oder nicht, eine vorab festgelegte Geometrie des Bandscheibenfachs mit ein und demselben Implantat zu erreichen. Die unterschiedlichen Abmessungen des Basisabschnitts sind dabei so ausgelegt, dass sie den Materialabtrag ausgleichen, der im Falle einer Bohrung entsteht und die Geometrie des Bandscheibenfachs zunächst verändern würde. Je nachdem, ob eine Bohrung erzeugt wird oder nicht, wird das Implantat in die erste oder in die zweite Winkelstellung überführt, sobald es seine axiale Sollposition im Bandscheibenfach erreicht hat.

Die Führungshülse kann an ihrem distalen Ende zwei Distanzzungen haben, die senkrecht zur Längsrichtung der Hülse eine Breite haben und die, wenn sich die Führungshülse mit der Schulter an zwei Wirbeln abstützt, das Bandscheibenfach in einer distrahierten Stellung halten, so dass in Abwesenheit des Implantats der Abstand zwischen den Wirbelkörpern gleich der Breite der Distanzzungen ist.

Um einen Ausgleich des Materials des Materialabtrags durch den Bohrer zu erzielen, sollte dann die Differenz zwischen den Abmessungen des Basisabschnitts in den zwei Richtungen gleich der Differenz zwischen dem vom Bohrkopf erzeugten Bohrungsdurchmesser und der Breite der Distanzzungen sein.

Stehen mehrere Bohrer zur Verfügung, die Bohrköpfe mit unterschiedlichen Durchmessern haben, oder mehrere Führungshülsen mit gleichem Innendurchmesser, aber unterschiedlich breiten Distanzzungen, zur Verfügung, so kann der Basisabschnitt auch in mehr als zwei Richtungen unterschiedliche Abmessungen haben. Mit einem Basisabschnitt, der im Querschnitt die Form eines unregelmäßigen Hexagons hat, lassen sich beispielsweise auch drei unterschiedliche Abstände einstellen.

Um schließlich das Implantat durch die Führungshülse in das Bandscheibenfach eindrehen zu können, kann das System ein Eindrehinstrument umfassen, an dem das Implantat lösbar befestigbar und durch die Führungshülse hindurch in das Bandscheibenfach eindrehbar ist. Ein Beispiel für ein geeignetes Eindrehinstrument ist in der am gleichen Tag eingereichten deutschen Patentanmeldung mit dem Titel "Instrumentarium zum Eindrehen eines Implantats in ein Bandscheibenfach" der Anmelderin beschrieben.

Gegenstand der Erfindung ist ferner ein Implantat-System für die Fusion von Wirbelkörpern, das ein (vorzugsweise mit einem Außengewinde versehenes) Implantat umfasst, das dazu eingerichtet ist, in ein von zwei Wirbeln begrenztes Bandscheibenfach eingedreht zu werden. Das Implantat hat an seinem proximalen, d.h. während des Eindrehvorgangs dem Chirurgen zugewandten, Ende einen Basisabschnitt, der entlang zweier Richtungen, die senkrecht zu der Längsachse des Implantats verlaufen, unterschiedliche Abmessungen hat. Das Implantat-System umfasst ferner eine Führungshülse, durch die hindurch das Implantat in das Bandscheibenfach eingedreht wird und an ihrem distalen Ende zwei Distanzzungen hat, die senkrecht zur Längsrichtung der Führungshülse eine Breite haben und die, wenn sich die Führungshülse mit der Schulter an den zwei Wirbeln abstützt, das Bandscheibenfach in einer distrahierten Stellung halten, so dass in Abwesenheit des Implantats der Abstand zwischen den Wirbelkörpern gleich der Breite der Distanzzungen ist. Ferner umfasst das Implantat-System einen Bohrer, der von einem proximalen, dem Chirurgen zugewandten Ende der Führungshülse her in diese einführbar ist und einen Schaft hat, der an seinem distalen, dem Chirurgen abgewandten Ende einen Bohrkopf trägt. Erfindungsgemäß sind die Abmessungen des Basisabschnitts entlang der zwei Richtungen derart an den Durchmesser des Bohrkopfes angepasst, dass dann, wenn mit dem Bohrer eine Bohrung erzeugt wurde und sich das Implantat in einer ersten Winkelstellung zwischen den Wirbelkörpern befindet, der Abstand und der Winkel zwischen den Wirbelkörpern gleich groß ist, wie wenn mit dem Bohrer keine Bohrung erzeugt wurde und sich das Implantat in einer zweiten Winkelstellung befindet, die durch Drehung aus der ersten Winkelstellung hervorgeht.

### KURZE BESCHREIBUNG DER ZEICHNUNGEN

Weitere Merkmale und Vorteile der Erfindung ergeben sich aus der nachfolgenden Beschreibung eines Ausführungsbeispiels anhand der Zeichnungen. Darin zeigen:
- Figur 1: eine perspektivische Ansicht einer Führungshülse, die eine Komponente des erfindungsgemäßen Systems darstellt;
- Figur 2: eine Vorderansicht der in der Figur 1 gezeigten Führungshülse;
- Figur 3: eine Seitenansicht der in der Figur 1 gezeigten Führungshülse;
- Figur 4: eine Untersicht der in der Figur 1 gezeigten Führungshülse;
- Figur 5: eine Seitenansicht eines Bohrers, der eine weitere Komponente des erfindungsgemäßen Systems ist;
- Figur 6: eine vergrößerte perspektivische Darstellung eines Implantats, das ebenfalls eine Komponente des erfindungsgemäßen Systems ist;
- Figur 7: eine Seitenansicht einer in das Bandscheibenfach eingeführten Führungshülse mit darin eingeführtem Bohrer;
- Figur 8: eine Seitenansicht wie Figur 7, jedoch mit aufgeschnittener Führungshülse;
- Figur 9: eine Seitenansicht des in das Bandscheibenfach eingedrehten Implantats in seiner axialen Sollposition und einer ersten Winkelstellung;
- Figur 10: das Implantat wie in der Figur 9, jedoch in einer zweiten Winkelstellung;
- Figur 11: eine Untersicht auf das in das Bandscheibenfach eingeführte Implantat in der ersten Winkelstellung;
- Figur 12: eine Untersicht auf das in das Bandscheibenfach eingeführte Implantat in der zweiten Winkelstellung.

### BESCHREIBUNG BEVORZUGTER AUSFÜHRUNGSBEISPIELE

### I. Aufbau

Das erfindungsgemäße Implantat-System umfasst ein Implantat, eine Führungshülse und einen Bohrer. Vorzugsweise sind diese Komponenten jeweils mehrfach, aber mit unterschiedlichen Abmessungen vorhanden, um unterschiedliche Winkel und Abstände zwischen den Wirbelkörpern einstellen zu können, die fusionieren sollen.

### a) Führungshülse

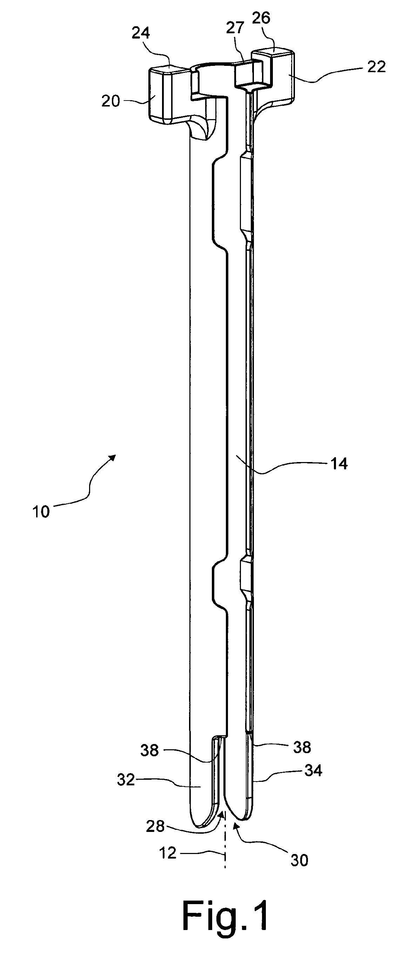

Der Aufbau einer der Führungshülsen wird im Folgenden mit Bezug auf die Figuren 1 bis 4 näher beschrieben. Diese Figuren zeigen die Führungshülse in einer perspektivischen Ansicht, einer Vorderansicht, einer Seitenansicht bzw. einer Untersicht.

Die insgesamt mit 10 bezeichnete Führungshülse hat ein proximales Ende, das in den Figuren nach oben weist und bei der Verwendung der Führungshülse 10 während einer Bandscheibenoperation dem Chirurgen zugewandt ist. Das gegenüberliegende, in den Figuren 1 bis 3 nach unten weisende Ende der Führungshülse 10 wird als distales Ende bezeichnet. Mit dem distalen Ende voran wird die Führungshülse 10 während der Operation durch eine vorab freigelegte Zugangsöffnung in den Patienten eingeführt.

Die Führungshülse 10 hat im Wesentlichen einen kreisringförmigen Querschnitt; möglich sind jedoch auch andere, z. B. quadratische, Querschnitte. Parallel zu einer Längsachse 12 der Führungshülse 10 erstreckt sich ein Längsschlitz 14, und zwar durchgehend vom proximalen Ende bis zum distalen Ende der Führungshülse 10.

Am proximalen Ende der Führungshülse 10 sind zwei Vorsprünge 20, 22 ausgebildet, die sich diametral einander gegenüberliegen und radial von der Längsachse 12 der Führungshülse 10 abstehen. Die proximalen Endflächen der beiden Vorsprünge 20, 22 bilden Einwirkflächen 24, 26, die eben sind und senkrecht zur Längsachse 12 der Führungshülse 10 verlaufen. Die Einwirkflächen 24, 26 können dazu verwendet werden, mit einem Hammer oder einem anderen Einschlaginstrument das distale Ende der Führungshülse 10 in das Bandscheibenfach zu treiben. Dies gelingt auch dann, wenn von dem proximalen Ende der Führungshülse 10 ein anderes Instrument, z. B. ein Bohrer oder ein Eindrehinstrument, hervorsteht, das zuvor in die Führungshülse 10 eingeführt wurde. Zwischen den Vorsprüngen 20, 22 erstreckt sich ein Steg 27, der in axialer Richtung etwas niedriger ist als die Vorsprünge 20, 22. Der Steg 27 dient in noch zu erläuternder Weise als Gegenfläche für einen am Bohrer ausgebildeten Anschlagsring.

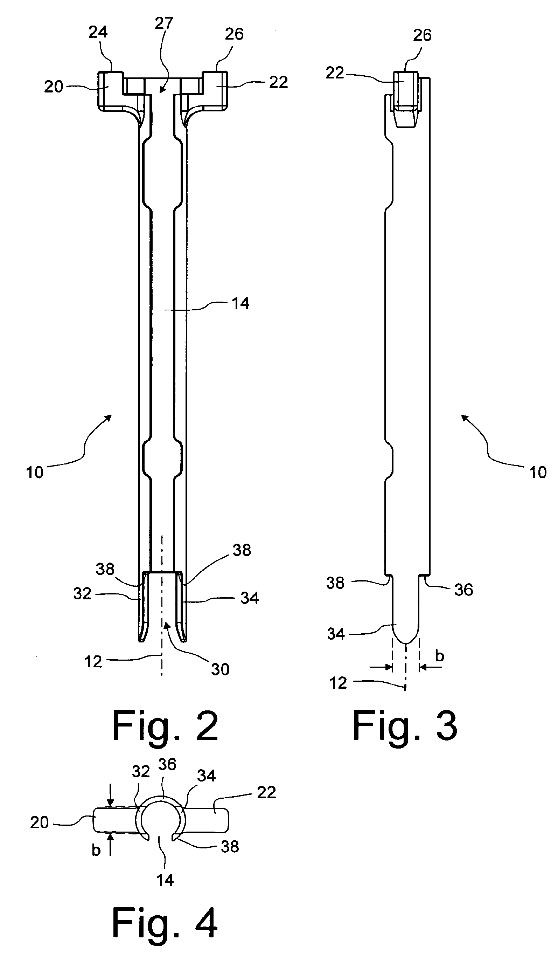

An ihrem distalen Ende weist die Führungshülse 10 zwei diametral einander gegenüberliegende Aussparungen 28, 30 auf, zwischen denen zwei Distanzzungen 32, 34 verbleiben, die einander gegenüberliegen. Die Distanzzungen 32, 34 werden somit durch einen Abschnitt der Führungshülsenwand gebildet, sind aber zum distalen Ende hin annähernd elliptisch abgerundet, wie man dies am besten in der Seitenansicht der Figur 3 erkennen kann.

Die proximale Stirnfläche der Aussparung 28 bildet eine erste Auflageschulter 36 (siehe Figuren 3 und 4), während die gegenüberliegende Aussparung 30 infolge des dort verlaufenden Längsschlitzes 14 zwei schmalere zweite Auflageschultern 38 festlegt.

Wie in den Figuren 3 und 4 erkennbar ist, haben die beiden Distanzzungen 32, 34 entlang einer Transversalrichtung, die senkrecht zur Längsachse 12 verläuft, eine Breite b. Wenn das System mehrere Führungshülsen 10 umfasst, so unterscheiden sich diese zumindest durch die Breiten b der Distanzzungen 32, 34 voneinander, ggf. aber auch durch den Innen- und Außendurchmesser der Führungshülsen 10.

### b) Bohrer

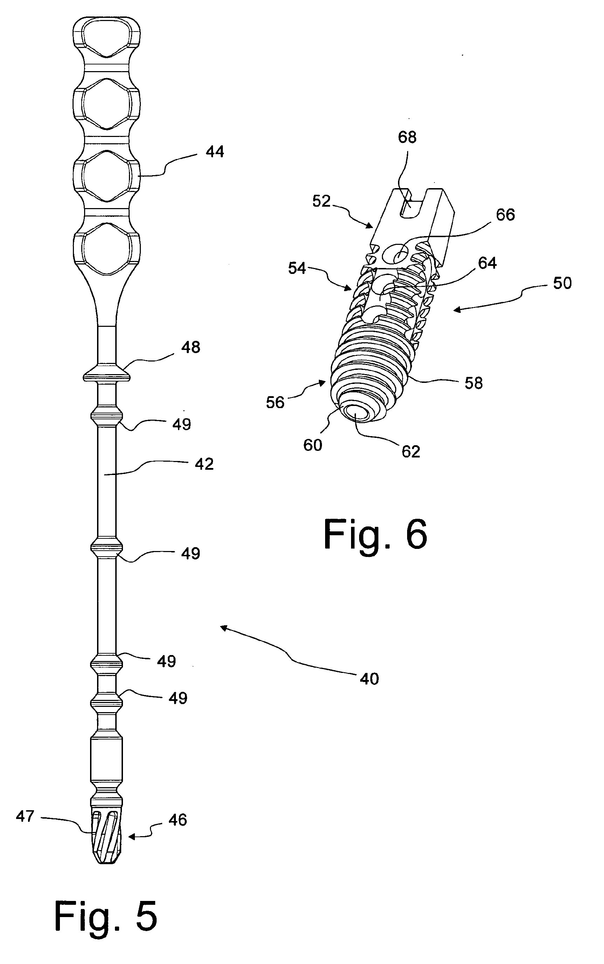

Die Figur 5 zeigt in einer Seitenansicht einen insgesamt mit 40 bezeichneten Bohrer, der ebenfalls eine Komponente des erfindungsgemäßen Systems ist. Der Bohrer hat einen im Wesentlichen stabförmigen Schaft 42, an dessen proximalem Ende ein Griff 44 angeformt ist. Am gegenüberliegenden distalen Ende trägt der Schaft 42 einen Bohrkopf 46, der in an sich bekannter Weise mit schräg verlaufenden Bohrschneiden 47 versehen ist.

Am Schaft 42 ist außerdem ein Anschlagsring 48 angeformt, dessen Durchmesser größer ist als der Innendurchmesser der Führungshülse 10.

Zwischen dem Anschlagsring 48 und dem Bohrkopf 46 ist der Schaft 42 mit mehreren ringförmigen Führungswülsten 49 versehen. Der Durchmesser der Führungswülste 49 ist etwas kleiner als der Innendurchmesser der Führungshülse 10, so dass der Bohrer 40 in der Führungshülse 10 geführt werden kann, ohne dabei zu verkippen.

### c) Implantat

Die Figur 6 zeigt in einer vergrößerten perspektivischen Darstellung von schräg vorne ein insgesamt mit 50 bezeichnetes Implantat, das ebenfalls eine Komponente des erfindungsgemäßen Systems ist. Das Implantat 50 hat in grober Näherung eine zylindrische Grundform, wobei allerdings die Umfangsflächen überwiegend leicht geneigt zu einer Längsachse des Implantats 50 verlaufen. Der größte Durchmesser liegt dabei in der vorderen, in der Figur 6 zum Betrachter weisenden Hälfte des Implantats 50, so dass sich die Grundform des Implantats 50 genauer als Doppelkegel beschreiben lässt.

Das Implantat hat einen Basisabschnitt 52, einen Zentralabschnitt 54 und einen Kopfabschnitt 56. Der Kopfabschnitt 56 läuft zum distalen, in der Figur 6 zum Betrachter weisenden Ende des Implantats 50 konisch zu und ist mit einem Außengewinde 58 versehen, das sich übergangslos am Zentralabschnitt 54 fortsetzt. Der dem distalen Ende unmittelbar benachbarte Teil des Kopfabschnitts 56 ist gewindefrei und bildet eine abgerundete Schutzkappe 60, die ringförmig eine Stirnöffnung 62 umgibt.

Der Zentralabschnitt 54 ist mit vier gleichmäßig über den Umfang verteilten und in axialer Richtung langgestreckten Fenstern 64 versehen, in denen das Außengewinde 58 unterbrochen ist und die jeweils mehrere kreisrunde Öffnungen 66 freigeben. Nachdem das Implantat 50 in das Bandscheibenfach eingesetzt wurde, kann Knochengewebe das Implantat 50 durch die Öffnungen 66 hindurch wachsen und auf diese Weise die gewünschte Fusion der benachbarten Wirbelkörper herbeiführen.

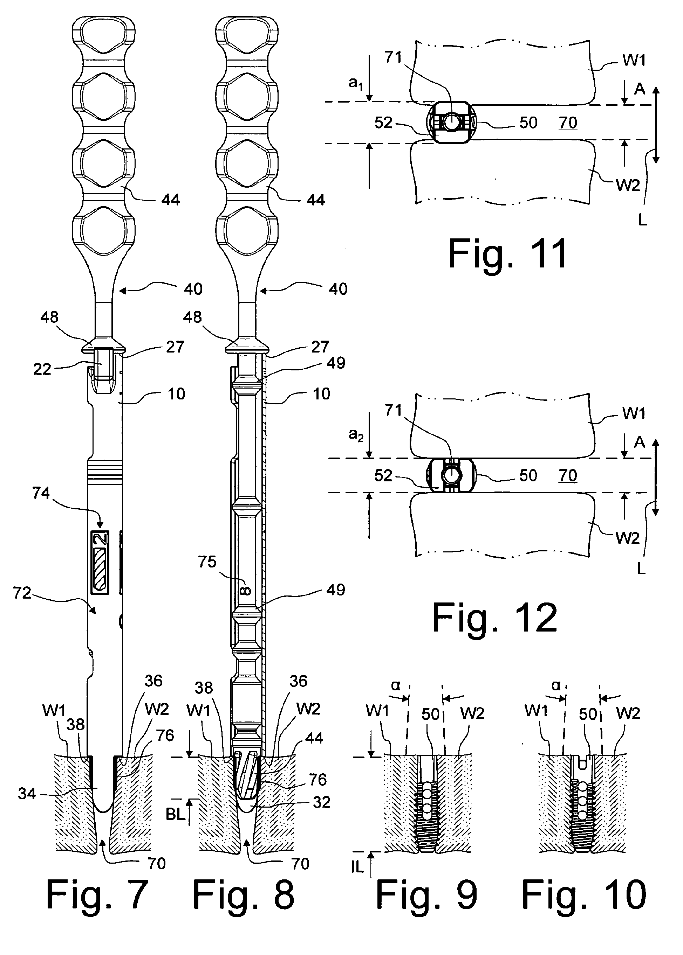

Der Basisabschnitt 52 ist im Wesentlichen als Vierflach ausgebildet, der allerdings in zwei zueinander orthogonalen Richtungen, die beide zur Längsrichtung des Implantats 50 orthogonal sind, unterschiedliche Abmessungen hat. Erkennbar ist dies gut in den Figuren 11 und 12, die das Implantat in zwei unterschiedlichen Winkelstellungen im implantierten Zustand zeigen. Die Breiten des Implantats 50 in diesen beiden Richtungen sind dort mit a₁ und a₂ bezeichnet.

Die beiden breiteren zueinander parallelen Planflächen, die zueinander den geringeren Abstand haben, sind jeweils mit einer Nut 68 versehen. In die Nuten 68 können Vorsprünge eines Eindrehinstruments formschlüssig eingreifen, um das Implantat 50 drehfest am Eindrehinstrument festzulegen. Im Inneren des Basisabschnitts 52 ist noch eine mit einem Innengewinde versehene Bohrung vorgesehen, mit der das Implantat 50 an dem Eindrehinstrument in axialer Richtung lösbar fixiert werden kann. In den Figuren 11 und 12 ist diese Bohrung erkennbar und mit 71 bezeichnet.

Weitere Einzelheiten zum Implantat sind der eingangs erwähnten US 2007/0055374 A1 entnehmbar.

### II. Verwendung

Um das Zusammenwirken der vorstehend in I. beschriebenen Komponenten verständlich zu machen, werden im Folgenden mit Bezug auf die Figuren 7 bis 12 einige der Schritte erläutert, die beim Einsetzen des Implantats 50 in ein Bandscheibenfach erforderlich sind.

In einem ersten Schritt wird festgelegt, welches von mehreren Implantaten 50 mit unterschiedlichen Abmessungen in das Bandscheibenfach eingesetzt werden soll. Diese Festlegung erfolgt meist auf der Grundlage von Funktionsaufnahmen des betroffenen Wirbelsäulensegments. Darunter versteht man eine Abfolge von Röntgenaufnahmen, in denen das betroffene Segment in unterschiedlichen Bewegungszuständen (z. B. Inklination, Reklination, Lateralflexion) gezeigt ist. Der Chirurg legt sodann fest, in welchem Abstand und in welchem Winkel zueinander die das Bandscheibenfach begrenzenden Wirbel nach dem Einsetzen des Implantats (im Allgemeinen werden allerdings zwei gleiche Implantate nebeneinander gesetzt) angeordnet sein sollen. Gegebenenfalls wird der behandelnde Chirurg hierbei durch ein Computerprogramm unterstützt, das eine Empfehlung für eine optimale Implantatgeometrie unter Berücksichtigung der biometrischen Besonderheiten des zu behandelnden Patienten ausgibt.

In einem zweiten Schritt wird ein Zugang zu dem Bandscheibenfach freigelegt. Dabei kann es sich beispielsweise um einen posterior-lateralen Zugang handeln. In den meisten Fällen wird dann die Bandscheibe ganz oder teilweise entfernt. Die das Bandscheibenfach begrenzenden Wirbelkörper bewegen sich dabei im Allgemeinen aufeinander zu, wodurch sich die Höhe des Bandscheibenfachs zunächst verringert.

In einem dritten Schritt wird das Bandscheibenfach distrahiert. Die Distraktion kann beispielsweise schrittweise unter Verwendung mehrerer unterschiedlich dimensionierter Führungshülsen und Distraktoren durchgeführt werden, wie dies in der oben bereits erwähnten unveröffentlichten deutschen Patentanmeldung mit dem Titel "System zum Distrahieren eines Bandscheibenfachs" beschrieben ist. Die letzte Führungshülse 10 ist dabei eine Führungshülse, die auf das einzusetzende Implantat 50 abgestimmt ist. Dies bedeutet, dass der Innendurchmesser der Führungshülse 10 geringfügig größer ist als der Außendurchmesser des zuvor ausgewählten Implantats 50. Nur dann lässt sich das Implantat mit Hilfe eines Eindrehinstruments durch die Führungshülse 10 schieben und in das Bandscheibenfach eindrehen.

Die Figuren 7 und 8 zeigen die Führungshülse 10 in einer Seitenansicht, wobei die Führungshülse 10 in der Figur 8 aufgeschnitten ist, um den darin geführten Bohrer 50 besser erkennen zu können. Die Führungshülse 10 ragt mit ihren Distanzzungen 32, 34 in ein mit 70 bezeichnetes Bandscheibenfach hinein, das von zwei im Schnitt dargestellten Wirbelkörpern W1, W2 begrenzt ist. Die Führungshülse 10 stützt sich dabei mit ihren Schultern 36, 38 an den Rändern der Wirbelkörper W1, W2 ab. Die Schultern 36, 38 dienen somit als Anschlag und verhindern zuverlässig ein weiteres Eindringen der Führungshülse 10 in das Bandscheibenfach 70.

Es sei hier beispielhaft angenommen, dass das Implantat einen maximalen Kerndurchmesser d_{K} = 8 mm und einen Außendurchmesser (d. h. unter Berücksichtigung des Außengewindes 58) d_{A}= 10 mm hat. Die Abmessungen des Basisabschnitts 52 betragen a₁ = 7 mm und a₂ = 5. Der Innendurchmesser der Führungshülse 10 ist, wie oben bereits erwähnt, geringfügig größer als der Außendurchmesser des Implantats 50. Auf der Führungshülse ist der Kerndurchmesser d_{K} des Implantats, für das die Führungshülse 10 geeignet ist, durch eine bei 72 eingravierte Ziffer (hier 8) angezeigt.

Wenn das Bandscheibenfach 70 auf 10 mm distrahiert werden kann, so wird nach Abschluss der Distraktion eine Führungshülse 10 eingesetzt, deren Distanzzungen 32, 34 eine Breite b= 10 mm haben. In diesem Fall könnte das Implantat 10 problemlos in das auf 10 mm distrahierte Bandscheibenfach 70 eingeführt werden, da sein Außendurchmesser d_{A} ja ebenfalls 10 mm beträgt.

Es kann jedoch vorkommen, dass die Distraktionsfähigkeit der das Bandscheibenfach 70 begrenzenden Wirbel W1, W2 nicht ausreicht, um das Bandscheibenfach 70 auf 10 mm zu distrahieren. Ursache hierfür kann z. B. ein besonders straffer Bänderapparat sein, der die Wirbelkörper W1, W2 umgibt. Um diesen Bänderapparat nicht zu schädigen, sollte die Distraktion der Wirbelkörper W1, W2 behutsam vorgenommen werden; eine Überdehnung ist zu vermeiden.

Im dargestellten Ausführungsbeispiel ist angenommen, dass eine Distraktion des Bandscheibenfachs 70 nur auf 8 mm möglich ist. Daher hat die nach Abschluss der Distraktion eingesetzte und in der Figur 7 in Seitenansicht gezeigte Führungshülse 10 Distanzzungen, deren Breite b lediglich 8 mm beträgt.

Damit trotzdem das Implantat mit einem Außendurchmesser d_{A} = 10 mm in das Bandscheibenfach eingedreht werden kann, könnte man erwägen, das Implantat sich mit seinem Außengewinde 58 in die proximalen, in den Figuren 7 und 8 nach oben weisenden Knochenränder der Wirbelkörper W1, W2 einschneiden zu lassen. Hierbei würden diese Knochenränder allerdings zerfurcht und dadurch destabilisiert. Zu berücksichtigen ist dabei, das in der axialen Sollposition des Implantats 50, wie sie in den Figuren 9 und 10 gezeigt ist, das Implantat nicht mit einem Gewindeabschnitt, sondern mit den Parallelflachs des Basisabschnitts 52 an diesen Knochenrändern anliegt. Um eine Destabilisierung der Knochenränder zu vermeiden, ist es günstiger, das Bandscheibenfach 70 mit Hilfe des Bohrers 40 auf 10 mm aufzubohren, so dass das Implantat 50 problemlos eingeführt werden kann.

Die Führungshülse 10 zeigt hierzu bei 74 an, um wie viele Millimeter der Abstand b der Distanzzungen 34, 36 kleiner ist als der Außendurchmesser d_{A} eines Implantats 50, dessen Kerndurchmesser d_{K} = 8 mm beträgt. Bei dem dargestellten Ausführungsbeispiel beträgt diese Differenz 2 mm. Folglich muss ein Bohrer 40 ausgewählt werden, dessen Bohrkopf 2 mm größer ist als der Kerndurchmesser (8 mm) des Implantats 50, mithin also ein Bohrer mit einem Bohrkopf 46, dessen Durchmesser 10 mm beträgt. In der Regel genügt es, wenn für jeden Kerndurchmesser d_{K} des Implantats 50 nur ein Bohrer 40 vorgesehen ist, der eine Bohrung mit dem Außendurchmesser d_{A} des Implantats 50 erzeugt. Daher braucht der Chirurg einfach nur den für den Kerndurchmesser d_{K} = 8 mm vorgesehenen Bohrer 40 (siehe Beschriftung bei 75 in Figur 8) zu verwenden.

Dieser Bohrer 46 wird nun in einem vierten Schritt mit seinem Bohrkopf 46 voran in die Führungshülse 10 eingeführt, bis der Bohrkopf 46 an den Knochenrändern der Wirbelkörper W1, W2 anschlägt. Durch Drehen des Griffs 44 wird dann der Bohrkopf 46 in Rotation um seine Längsachse versetzt und auf diese Weise spanabhebend eine Bohrung mit einem Durchmesser von 10 mm erzeugt. Diese Bohrung ist groß genug, um das Implantat 50 anschließend in die Bohrung einführen zu können. Der Bohrkopf 46 entfernt während des Bohrens Knochenmaterial, das in den Figuren 7 und 8 durch dicke schwarze Linien 76 zu beiden Seiten der dem Betrachter zugewandten Distanzzunge 34 angedeutet ist.

Während des Bohrvorgangs dringt der Bohrkopf 46 immer weiter in das Bandscheibenfach 70 ein. Dieser Eindringvorgang endet in dem Moment, an dem der am Schaft 42 des Bohrers 40 angeformte Anschlagsring 48 an dem Steg 27 der Führungshülse 10 anschlägt. Wie man in der Figur 8, in der die Führungshülse 10 aufgeschnitten ist, gut erkennen kann, reicht in dieser axialen Anschlagsposition der Bohrkopf 46 des Bohrers 40 nur ein relativ kurzes Stück in das Bandscheibenfach 70 hinein. Die mit BL angedeutete Bohrlänge des Bohrers 40 ist dabei sogar noch geringer als die axiale Länge der Distanzzungen 32, 34. Insbesondere ist die Bohrlänge BL vor allem jedoch deutlich kleiner als die in der Figur 9 erkennbare Implantatlänge IL des Implantats 50. Die relativ kleine Bohrlänge BL genügt jedoch, um das Implantat 50 mit dem vergleichsweise dicken Kopfabschnitt 56 voran in das Bandscheibenfach 70 einführen zu können.

Auch eine kurze Bohrlänge BL verändert jedoch die Geometrie des Bandscheibenfachs 70 auf der proximalen, in den Figuren 7 und 8 nach oben weisenden Seite. Um hierfür einen Ausgleich zu schaffen, sind die unterschiedlichen Abmessungen a₁, a₂ des Basisabschnitts 52 des Implantats 50 so an den Durchmesser des Bohrkopfes 46 angepasst, dass dann, wenn mit dem Bohrer 40 eine Bohrung erzeugt wurde und sich das Implantat 50 in einer ersten Winkelstellung zwischen den Wirbelkörpern W1 und W2 befindet, der Abstand und der Winkel zwischen den Wirbelkörpern W1, W2 gleich groß ist, wie wenn mit dem Bohrer 40 keine Bohrung erzeugt worden wäre und sich das Implantat 50 in einer zweiten Winkelstellung befindet, die hier durch Drehung um 90° aus der ersten Winkelstellung hervorgeht.

Dies ist in den Figuren 11 und 12 gezeigt, die eine Untersicht des Implantats 50 in der ersten bzw. in der zweiten Winkelstellung zeigen, nachdem das Implantat 50 vollständig in das Bandscheibenfach 70 eingedreht wurde. In der in der Figur 11 gezeigten ersten Winkelstellung ist der Basisabschnitt 52 des Implantats 50 so orientiert, dass sich die Seite mit der größeren Breite a₁ parallel zu einer mit einem Doppelpfeil angedeuteten Richtung L erstreckt, die annähernd parallel zur Längsachse der Wirbelsäule des Patienten verläuft. Wegen der vorher durchgeführten Bohrung taucht der Basisabschnitt 52 des Implantats 50 in dieser ersten Winkelstellung ein wenig in die Wirbelkörper W1, W2 ein. Da die Bohrung einen Durchmesser hat, der 2 mm größer ist als die Distraktion des Bandscheibenfachs 70, beträgt diese Eintauchtiefe an jedem der Wirbelkörper W1, W2 jeweils 1 mm.

Wird hingegen auf eine Bohrung verzichtet, weil das Bandscheibenfach 70 zuvor auf 10 mm distrahiert werden konnte, so wird das Implantat 50 in der in der Figur 12 gezeigten zweiten Winkelstellung eingesetzt, die durch Drehung um 90° aus der in der Figur 11 gezeigten ersten Winkelstellung hervorgeht. In dieser zweiten Winkelstellung ist der Basisabschnitt 52 des Implantats 50 so orientiert, dass sich die Seite mit der kleineren Breite a₂ parallel zu der Richtung L erstreckt. Die Differenz der beiden Breiten a₁- a₂ = 2 mm gleicht somit genau den Materialabtrag aus, der durch den Bohrer 40 beim Aufbohrens des Bandscheibenfachs 70 in den benachbarten Wirbelkörpern W1, W2 bei 76 erzeugt wurde.

Folglich ist der in den Figuren 11 und 12 mit A bezeichnete Abstand zwischen den Wirbelkörpern W1, W2 am proximalen Ende in beiden Winkelstellungen des Implantats 50 identisch, nämlich a₂ = 5 mm.

Entsprechendes gilt auch für den Winkel α zwischen den Wirbelkörpern W1, W2. Hierzu wird ergänzend auf die Figur 10 verwiesen, in der das Implantat 50 im Vergleich zu der in der Figur 9 gezeigten zweiten Winkelstellung in der ersten Winkelstellung im Bandscheibenfach 70 angeordnet ist. Der Winkel α zwischen den beiden das Bandscheibenfach 70 begrenzenden Wirbeln W1, W2 ist in beiden Fällen identische und hängt somit nicht davon ab, ob zuvor das Bandscheibenfach 70 aufgebohrt wurde oder nicht.

Da somit der Abstand A und auch der Winkel α zwischen den Wirbeln W1, W2 identisch ist unabhängig davon, ob vorher eine Bohrung erzeugt wurde oder nicht, wird in jedem Fall die gleiche vorab festgelegte Geometrie des Bandscheibenfachs 70 durch das Implantat 50 eingestellt.

Durch die Verdrehung des Implantats 50 um 90° zum Wechseln zwischen der ersten und der zweiten Winkelstellung wird im Prinzip auch die Axialposition des Implantats 50 im Bandscheibenfach 70 geringfügig verändert. Infolge der annähernd doppelkonischen Form des Implantats 50 wirkt sich dies auch auf die Geometrie des Bandscheibenfachs 70 aus. Die Steigung des Außengewindes 58 des Implantats 50 ist jedoch so gering, dass der axiale Versatz des Implantats 70 bei einer Drehung um 90° deutlich kleiner als 1 mm ist. Die damit einhergehende Veränderung der Geometrie des Bandscheibenfachs 70 ist deswegen vernachlässigbar.

## Patentansprüche

1. Implantat-System für die Fusion von Wirbelkörpern, mit:
a) einem Implantat (50), das entlang einer Längsachse eine Implantatlänge (IL) hat und dazu eingerichtet ist, in ein von zwei Wirbeln (W1, W2) begrenztes Bandscheibenfach (70) eingedreht zu werden,
b) einer Führungshülse (10), durch die hindurch das Implantat (50) in das Bandscheibenfach (70) eingedreht wird und die sich während des Eindrehvorgangs mit einer Schulter (36, 38) an den zwei Wirbeln (W1, W2) abstützt, und mit
c) einem Bohrer (40), der von einem proximalen, dem Chirurgen zugewandten Ende der Führungshülse (10) her in diese einführbar ist und einen Schaft (42) hat, der an seinem distalen, dem Chirurgen abgewandten Ende einen Bohrkopf (46) trägt,
**dadurch gekennzeichnet, dass**
an dem Schaft (42) des Bohrers (40) ein Anschlag (48) ausgebildet ist, der mit der Führungshülse (10) derart zusammenwirkt, dass ein weiteres Einführen des Bohrers (40) in die Führungshülse (10) verhindert wird, sobald der Bohrer (40) relativ zu der Führungshülse (10) eine axiale Anschlagsposition erreicht, und dass
der Bohrkopf (46) in der axialen Anschlagsposition um eine Bohrlänge (BL) über die Schulter (36, 38) der Führungshülse (10) hinausragt, die kleiner ist als die Implantatlänge (IL).

2. Implantat-System nach Anspruch 1, **dadurch gekennzeichnet, dass** das Implantat (50) seinen größten Durchmesser in seiner distalen, d. h. während des Eindrehvorgangs vom Chirurgen abgewandten, Hälfte hat.

3. Implantat-System nach Anspruch 2, **dadurch gekennzeichnet, dass** das Implantat (50) im Wesentlichen die Grundform eines Doppelkegels hat.

4. Implantat-System nach einem der vorhergehenden Ansprüche, **dadurch gekennzeichnet, dass** die Bohrlänge (BL) kleiner als 2/3 der Implantatlänge (IL) ist.

5. Implantat-System nach Anspruch 4, **dadurch gekennzeichnet, dass** die Bohrlänge (BL) kleiner als 1/2 der Implantatlänge (IL) ist.

6. Implantat-System nach einem der vorhergehenden Ansprüche, **dadurch gekennzeichnet, dass** das Implantat (50) an seinem proximalen, d.h. während des Eindrehvorgangs dem Chirurgen zugewandten, Ende einen Basisabschnitt (52) hat, der entlang zweier Richtungen, die senkrecht zu der Längsachse des Implantats verlaufen, unterschiedliche Abmessungen (a₁, a₂) hat.

7. Implantat-System nach Anspruch 6, **dadurch gekennzeichnet, dass** die Abmessungen (a₁, a₂) entlang der zwei Richtungen derart an den Durchmesser des Bohrkopfes (46) angepasst sind, dass dann, wenn mit dem Bohrer (40) eine Bohrung erzeugt wurde und sich das Implantat (50) in einer ersten Winkelstellung zwischen den Wirbelkörpern (W1, W2) befindet, der Abstand (A) und der Winkel (α) zwischen den Wirbelkörpern (W1, W2) gleich groß ist, wie wenn mit dem Bohrer (40) keine Bohrung erzeugt wurde und sich das Implantat (50) in einer zweiten Winkelstellung befindet, die durch Drehung aus der ersten Winkelstellung hervorgeht.

8. Implantat-System nach einem der vorhergehenden Ansprüche, **dadurch gekennzeichnet, dass** die Führungshülse (10) an ihrem distalen Ende zwei Distanzzungen (32, 34) hat, die senkrecht zur Längsrichtung (12) der Hülse (10) eine Breite (b) haben und die, wenn sich die Führungshülse (10) mit der Schulter (36, 38) an den zwei Wirbeln (W1, W2) abstützt, das Bandscheibenfach (70) in einer distrahierten Stellung halten, so dass in Abwesenheit des Implantats (50) der Abstand zwischen den Wirbelkörpern (W1, W2) gleich der Breite (b) der Distanzzungen (32, 34) ist.

9. Implantat-System nach Anspruch 8 bei Rückbezug auf den Anspruch 7, **dadurch gekennzeichnet, dass** die Differenz zwischen den Abmessungen (a₁, a₂) des Basisabschnitts (52) in den zwei Richtungen gleich ist der Differenz zwischen dem vom Bohrkopf erzeugten Bohrungsdurchmesser und der Breite der Distanzzungen (b).
